# EUROPÄISCHE PATENTANMELDUNG

(11) **EP 1 570 811 A1**
(43) Veröffentlichungstag der Anmeldung: **07.09.2005**
(21) Anmeldenummer: 04005020.5
(22) Anmeldetag: 03.03.2004
(51) Int. Cl.: A61F 2/36, A61B 17/16

(54) **Fermurkopfprothese**

(71) Anmelder: Centerpulse Orthopedics Ltd., 6340 Baar (CH)
(72) Erfinder: Ganz, Reinhold, 3073 Gümligen/Bern (CH); Marchione, Andreas, 8404 Winterthur (CH); Willi, Roland, 8413 Neftenbach (CH)
(74) Vertreter: Manitz, Finsterwald & Partner GbR

(57) **Zusammenfassung**

Die Erfindung betrifft eine Femurkopfprothese mit einer sphärischen Kappe (1) und einem im zusammengesetzten Zustand in der Kappe abgestützten Stift (3), welcher über die Kappe zur Verankerung im Knochengewebe vorsteht, wobei der Stift mit einem Kopfteil (7) um seine Achse drehbar, jedoch in axialer und radialer Richtung gefangen und mit einem Werkzeug durch eine Öffnung (17) im Polbereich der Kappe erreichbar ist.

## Beschreibung

Die Erfindung betrifft eine Femurkopfprothese mit einer sphärischen Kappe und einem im zusammengesetzten Zustand in der Kappe abgestützten Stift, welcher über die Kappe zur Verankerung im Knochengewebe vorsteht.

Femurkopfprothesen der eingangs genannten Art sind grundsätzlich bekannt. Bei Verwendung einer derartigen Femurkopfprothese kann der natürliche Femurkopf des Patienten weitgehend erhalten werden. Der Femurkopf wird bei der Operation lediglich an seiner Oberfläche bearbeitet, um das Aufsetzen der sphärischen Kappe zu ermöglichen. Derartige Femurkopfprothesen sind insbesondere für Patienten mittleren Alters und mit guter Knochenqualität geeignet.

Der Erfindung liegt die Aufgabe zugrunde, eine Femurkopfprothese der eingangs genannten Art zu schaffen, die sich durch einen einfachen Aufbau bei gleichzeitig sicherer und zuverlässiger Funktionsweise auszeichnet und insbesondere beim Einsetzen in das Hüftgelenk des Patienten einfach zu handhaben ist.

Die Lösung dieser Aufgabe erfolgt durch die Merkmale des Anspruchs 1 und insbesondere dadurch, dass der Stift mit einem Kopfteil um seine Achse drehbar, jedoch in axialer und radialer Richtung gefangen und mit einem Werkzeug durch eine Öffnung im Polbereich der Kappe erreichbar ist.

Bei der erfindungsgemäßen Femurkopfprothese ist vorgesehen, dass der Stift um seine Längsachse drehbewegbar, hinsichtlich einer Bewegung in axialer und radialer Richtung relativ zu der sphärischen Kappe jedoch eingeschränkt ist. Dies wird dadurch erreicht, dass der Stift mit einem Kopfteil an der Kappe gefangen und somit unverlierbar an der Kappe gehalten ist. Das Kopfteil ist durch eine Öffnung im Polbereich der Kappe zugänglich, so dass der Stift mittels eines Werkzeuges in eine Drehbewegung versetzt werden kann. Die Drehbewegung des Stifts wird insbesondere dadurch ermöglicht, dass die mechanische Verbindung zwischen dem Stift und der sphärischen Kappe mit Spiel behaftet ist.

Die erfindungsgemäße Femurkopfprothese besitzt den Vorteil, dass sie bereits vor dem Einsetzen in das Hüftgelenk des Patienten zusammengesetzt ist. Hierdurch kann die sphärische Kappe gewissermaßen automatisch mit der Verankerung des Stifts auf den natürlichen Femurkopf aufgesetzt werden. Ein separates Aufsetzen und zwischenzeitliches Halten der sphärischen Kappe durch den Operateur entfällt. Die erfindungsgemäße Femurkopfprothese ist folglich besonders einfach zu handhaben. Im weiteren ist gewährleistet, dass der Stift nie soweit aus der Kappe vorstehen kann, dass die Hüftgelenkschale durch den Stift verletzt wird.

Vorteilhafte Ausführungsformen der Erfindung sind in den Unteransprüchen, der Beschreibung sowie der Zeichnung angegeben.

Vorzugsweise ist eine äußere Berührungsfläche des Stifts zum Knochenmaterial nicht-rotationssymmetrisch ausgeführt. Insbesondere kann der Stift als Knochenschraube mit einem Gewinde ausgeführt sein, so dass der Stift in den natürlichen Kugelkopf einschraubbar ist. Hierdurch kann eine besonders feste und sichere Verankerung der Femurkopfprothese gewährleistet und eine Lockerung des künstlichen Hüftgelenks weitgehend vermieden werden.

Ein besonders einfacher Aufbau der erfindungsgemäßen Femurkopfprothese ergibt sich, wenn gemäß einem Ausführungsbeispiel der Erfindung das Kopfteil einen Kragen zur axialen und radialen Halterung aufweist. Der Kragen kann bezüglich der Längsachse des Stifts rotationssymmetrisch ausgeführt sein.

Des Weiteren kann das Kopfteil einen Innensechskant für ein Schraubwerkzeug aufweisen, so dass der Stift z.B. mit einem herkömmlichen Inbusschlüssel in den natürlichen Femurkopf eingedreht bzw. eingeschraubt werden kann.

Ferner kann erfindungsgemäß vorgesehen sein, dass das Kopfteil durch einen in die Kappe eingesetzten Sicherungsring gefangen ist. Beim Zusammenbau der Femurkopfprothese kann der Stift zunächst in die Kappe eingesetzt und anschließend durch den ebenfalls in die Kappe eingesetzten Sicherungsring gesichert werden.

Um zu gewährleisten, dass beim Zusammenbau der Femurkopfprothese der Stift korrekt in die Kappe eingesetzt wird, kann die Innenseite der Kappe im Polbereich eine Vertiefung aufweisen, in die das Kopfteil des Stifts zumindest teilweise eingesetzt ist.

Es ist weiterhin bevorzugt, dass die Innenseite der Kappe einen sphärischen Bereich und einen anschließenden zylindrischen Bereich aufweist. Durch das Nachformen der sphärischen Geometrie der Oberfläche des Femurkopfs kann gewährleistet werden, dass beim Bearbeiten des natürlichen Femurkopfs möglichst wenig natürliches Knochenmaterial entfernt werden muss. Der sphärische Bereich kann insbesondere an der Äquatoriallinie der Kappe in den zylindrischen Bereich übergehen.

Besonders vorteilhaft ist es, wenn die Kappe im Randbereich wenigstens eine sich über einen Teil des Umfangs erstreckende Aussparung aufweist. Die sphärische Kappe wird vorteilhafterweise derart auf dem natürlichen Kugelkopf aufgesetzt, dass die Aussparung mit der Arterie Ramus Acetabularis des natürlichen Kugelkopfs zusammenfällt. Hierdurch wird gewährleistet, dass diese nicht entfernt werden muss und der proximale Teil des natürlichen Femurkopfs weiterhin mit Blut versorgt werden kann.

Nach einer weiteren Ausgestaltung der Erfindung weist ein als Knochenschraube ausgebildeter Stift an seiner Spitze einen Zapfen auf. Hierdurch wird das Einsetzen der Knochenschraube in eine im natürlichen Kugelkopf vorgesehene Bohrung erleichtert.

Kappe und Stift können aus Metall gefertigt sein. Die Innenseite der Kappe ist bevorzugt mit Titan oder einer Titanlegierung beschichtet, so dass der natürliche Kugelkopf mit einem körperverträglichen Material in Verbindung steht.

Die Erfindung betrifft außerdem ein Fräswerkzeug zur Femurkopfbearbeitung für eine Femurkopfprothese, wie sie vorstehend erläutert ist, mit einem zentralen Führungsstift und wenigstens einer Schneide, welche zumindest bereichsweise entsprechend der Innenseite des sphärischen Bereichs der Kappe gekrümmt ist.

Insbesondere können mindestens zwei, insbesondere wenigstens drei zueinander in Drehrichtung versetzte Schneiden vorgesehen sein, die an ihren äußeren Enden über einen Ring miteinander verbunden sind.

Die Erfindung betrifft weiterhin einen Schneidstempel zur Femurkopfbearbeitung für eine Femurkopfprothese, wie sie vorstehend erläutert ist, mit einer ringförmigen Schneide vom Innendurchmesser eines Randbereichs der Kappe, wobei die Schneide einen bereichsweisen Unterbruch aufweist, welcher einer Aussparung im Randbereich der Kappe entspricht.

Die Verwendung dieser Werkzeuge wird an anderer Stelle näher erläutert.

Die Erfindung wird im Folgenden beispielhaft unter Bezugnahme auf die Zeichnung beschrieben. Es zeigen:
- Fig. 1: eine erfindungsgemäße Femurkopfprothese im Querschnitt, und
- Fig. 2: eine Seitenansicht einer erfindungsgemäßen Femurkopfprothese.

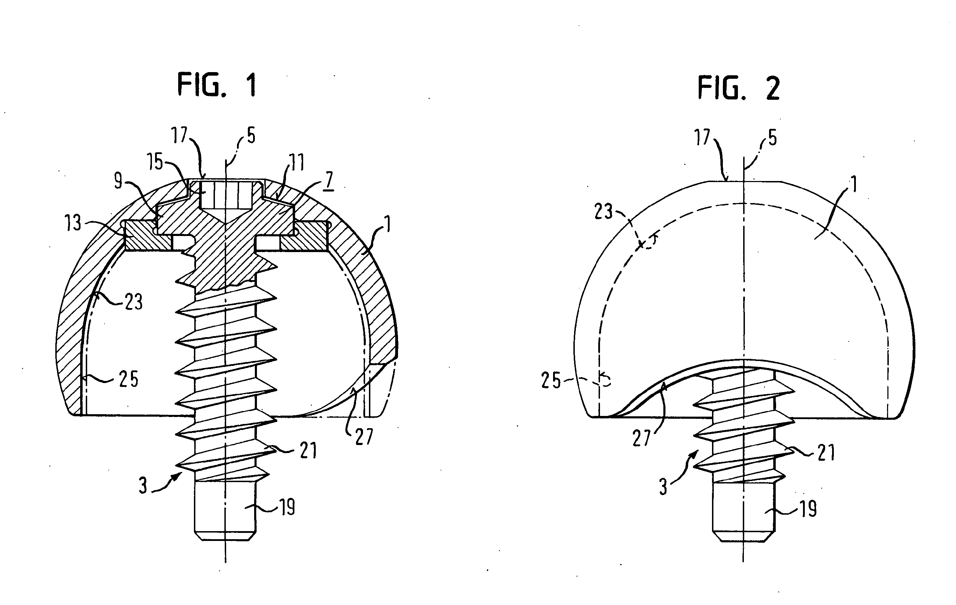

Fig. 1 zeigt eine im zusammengesetzten Zustand dargestellte erfindungsgemäße Femurkopfprothese mit einer sphärischen Kappe 1 und einer lang gestreckten Knochenschraube 3, die entlang einer Polachse 5 der sphärischen Kappe 1 angeordnet ist, so dass die Polachse 5 der sphärischen Kappe 1 und die Längsachse 5 der Knochenschraube 3 zusammenfallen. Im zusammengesetzten Zustand steht die Knochenschraube 3 über das dem Pol entgegengesetzte offene Ende der Kappe 1 hervor.

Die Knochenschraube 3 umfasst ein Kopfteil 7, das einen Kragen 9 mit kreisförmigen Querschnitt aufweist, der in eine entsprechend geformte, im Polbereich der Kappe 1 ausgebildete Vertiefung 11 an der Innenseite der Kappe 1 eingesetzt ist. Der Kragen 9 wird dabei durch einen in die Kappe 1 eingesetzten Sicherungsring 13 gesichert. Die Vertiefung 11 und der Sicherungsring 13 sind derart ausgebildet, dass der Kragen 9 radial und axial mit etwas Spiel behaftet an der sphärischen Kappe 1 gehalten ist, so dass die Knochenschraube 3 um ihre Längsachse 5 relativ zu der sphärischen Kappe 1 gedreht werden kann und gleichzeitig unverlierbar mit der Kappe 1 verbunden ist.

Das durch die Vertiefung 11 und den Sicherungsring 13 gefangene Kopfteil 7 umfasst eine als Innensechskant 15 ausgebildete Ausnehmung, in die ein Sechskantschlüssel zum Drehen der Knochenschraube 3 eingreifen kann. Hierzu ist im Polbereich der sphärischen Kappe 1 eine Öffnung 17 ausgebildet, so dass das Kopfteil 7 der Knochenschraube 3 für den Sechskantschlüssel zugänglich ist.

Das dem Kopfteil 7 entgegengesetzte Ende der Knochenschraube 3 weist einen im Wesentlichen zylinderförmig ausgebildeten gewindefreien Zapfen 19 auf. Der zwischen dem Kopfteil 7 und dem Zapfen 19 befindliche Teil der Knochenschraube 3 ist mit einem Gewinde 21 versehen, so dass eine äußere Berührungsfläche der Knochenschraube 3 zum Knochenmaterial nicht-rotationssymmetrisch ausgeführt ist.

Die Innenseite der Kappe 1 weist einen sich über einen Winkelbereich von 180° erstreckenden sphärischen Bereich 23 und einen daran anschließenden zylindrischen Bereich 25 auf. Die Außenseite der Kappe 1 ist abgesehen von der im Polbereich ausgebildeten Öffnung 17 und einer nachstehend noch näher erläuterten Aussparung unterbrechungsfrei sphärisch ausgebildet. Die Kappe 1 als Ganzes weist im Wesentlichen die Form einer Halbkugelschale mit einem unten angesetzten Zylinderring auf, dessen Höhe kleiner als der Radius der Halbkugel ist. Die Innenseite der Kappe 1 ist mit Titan oder einer Titanlegierung beschichtet.

Wie sich insbesondere aus Fig. 2 ergibt, weist die Kappe 1 im Randbereich wenigstens eine sich über einen Teil des Umfangs erstreckende Aussparung 27 auf, die ermöglicht, dass die Arterie Ramus Acetabularis des natürlichen Kugelkopfs trotz aufgesetzter Femurkopfprothese nicht entfernt werden muss.

Nachfolgend wird das Anbringen einer erfindungsgemäßen Femurkopfprothese an einen natürlichen Femurkopf unter teilweiser Bezugnahme auf die Figuren beschrieben.

Zunächst wird ein Zielgerät, das nach Art einer C-förmigen, verstellbaren Klemmvorrichtung ausgebildet ist und das Setzen eines Führungselements, insbesondere eines Steinmann-Nagels, erlaubt, an die geplante laterale Perforationsstelle des Femurs angesetzt und mittels einer konischen Kappe grob am Femurkopf ausgerichtet und fixiert.

Danach wird mittels eines Abtastarms die Position einer Führung des Zielgeräts am Femurkopf überprüft und korrigiert. Ist die gewünschte Position erreicht, wird der Steinmann-Nagel gesetzt, wobei Position und Orientierung des Steinmann-Nagels durch die Führung des Zielgeräts vorgegeben sind. Anschließend wird das Zielgerät entfernt und der Steinmann-Nagel bis zur lateralen Perforation gesetzt.

Als nächstes wird auf den Femurkopf ein Fräswerkzeug aufgesetzt. Der Steinmann-Nagel dient zur Ausrichtung des Fräswerkzeugs, das über einen hülsenartigen zentralen Führungsstift durch den Steinmann-Nagel geführt wird. Das Fräswerkzeug umfasst weiterhin drei zueinander in Drehrichtung versetzte Schneiden, die entsprechend der Innenseite des sphärischen Bereichs der erfindungsgemäßen Kappe 1 (Fig. 1, 2) gekrümmt sind. Zur gegenseitigen Stabilisierung sind die Schneiden an ihren äußeren Enden über einen Ring miteinander verbunden.

Mittels des Fräswerkzeugs wird dann die Oberfläche des natürlichen Kugelkopfs entsprechend der Form der Innenseite der sphärischen Kappe abgefräst, so dass die Femurkopfprothese möglichst formschlüssig an den natürlichen Kugelkopf angebracht werden kann. Insbesondere wird durch das Fräswerkzeug der bei aufgesetzter Femurkopfprothese an den Sicherungsring 13 angrenzende Polbereich des natürlichen Kugelkopfs abgeflacht.

Nachdem das Fräswerkzeug entfernt wurde, wird ein ebenfalls durch den Steinmann-Nagel geführter Schneidstempel aufgesetzt, welcher eine ringförmige Schneide vom Innendurchmesser des Randbereichs der erfindungsgemäßen Kappe 1 aufweist. Der Schneidstempel wird ähnlich einem Stemmeisen oder Meißel mit Druck, beispielsweise durch einen Gleithammer, beaufschlagt, so dass über den abgefrästen Bereich des natürlichen Kugelkopfs radial hervorstehendes Knochenmaterial zumindest der Höhe des Zylinderrings entsprechend entfernt und dadurch eine Anpassung an den zylindrischen Abschnitt 25 der Kappe 1 geschaffen wird. Die Schneide weist einen bereichsweisen Unterbruch auf, welcher im Wesentlichen der Aussparung des Randbereichs der erfindungsgemäßen Kappe 1 entspricht, so dass ein Durchtrennen der Arterie Ramus Acetabularis vermieden werden kann.

Daraufhin wird mittels eines durch den Steinmann-Nagel geführten Bohrers eine Öffnung in den natürlichen Femurkopf zur Aufnahme der erfindungsgemäßen Knochenschraube 3 gebohrt.

Anschließend wird eine Testkappe aufgesetzt, die eine der endgültig einsetzbaren Femurkopfprothese entsprechende Geometrie aufweist und aus der ein Fenster ausgeschnitten ist, so dass das möglichst passgenaue Anliegen der Innenseite der Kappe an dem bearbeiteten natürlichen Femurkopf überprüft werden kann. Zusätzlich wird die korrekte Lage der Testkappe insbesondere in axialer Richtung an dem Steinmann-Nagel markiert.

Ist die Oberfläche des natürlichen Kugelkopfs der Innenseite der sphärischen Kappe 1 korrekt nachgeformt worden, wird die zur Aufnahme der Knochenschraube 3 zuvor gebohrte Öffnung mittels eines durch den Steinmann-Nagel geführten Gewindeschneiders mit einem Innengewinde versehen.

Schließlich wird der Steinmann-Nagel entfernt und die erfindungsgemäße Femurkopfprothese (Fig. 1, 2) auf dem wie vorstehend erläutert bearbeiteten Kugelkopf aufgesetzt. Die Knochenschraube 3 wird eingeschraubt, bis das Anzugsmoment der Schraube merklich ansteigt. Dann wird mittels der Markierung überprüft, ob die Femurkopfprothese ihre korrekte Lage einnimmt. Die sphärische Kappe 1 wird nun derart um ihre Polachse 5 gedreht, dass die Aussparung 27 mit der Arterie Ramus Acetabularis zusammenfällt. Danach wird die Knochenschraube 3 festgezogen und die erfindungsgemäße Femurkopfprothese in ihrer Endposition fixiert.

### Bezugszeichenliste

- 1: sphärische Kappe
- 3: Knochenschraube
- 5: Polachse, Längsachse
- 7: Kopfteil
- 9: Kragen
- 11: Vertiefung
- 13: Sicherungsring
- 15: Innensechskant
- 17: Öffnung
- 19: Zapfen
- 21: Gewinde
- 23: sphärischer Bereich
- 25: zylindrischer Bereich
- 27: Aussparung

## Patentansprüche

1. Femurkopfprothese mit einer sphärischen Kappe (1) und einem im zusammengesetzen Zustand in der Kappe (1) abgestützten Stift (3), welcher über die Kappe (1) zur Verankerung im Knochengewebe vorsteht,
**dadurch gekennzeichnet,**
**dass** der Stift (3) mit einem Kopfteil (7) um seine Achse (5) drehbar, jedoch in axialer und radialer Richtung gefangen und mit einem Werkzeug durch eine Öffnung (17) im Polbereich der Kappe (1) erreichbar ist.

2. Femurkopfprothese nach Anspruch 1,
**dadurch gekennzeichnet,**
**dass** eine äußere Berührungsfläche des Stifts (3) zum Knochenmaterial nicht-rotationssymmetrisch ausgeführt ist.

3. Femurkopfprothese nach Anspruch 1 oder 2,
**dadurch gekennzeichnet,**
**dass** der Stift als Knochenschraube (3) ausgeführt ist.

4. Femurkopfprothese nach einem der Ansprüche 1 bis 3,
**dadurch gekennzeichnet,**
**dass** das Kopfteil (7) einen Kragen (9) zur axialen und radialen Halterung aufweist.

5. Femurkopfprothese nach einem der Ansprüche 1 bis 4,
**dadurch gekennzeichnet,**
**dass** das Kopfteil (7) einen Innensechskant (15) für ein Schraubwerkzeug aufweist.

6. Femurkopfprothese nach einem der Ansprüche 1 bis 5,
**dadurch gekennzeichnet,**
**dass** das Kopfteil (7) durch einen in die Kappe (1) eingesetzten Sicherungsring (13) gefangen ist.

7. Femurkopfprothese nach einem der Ansprüche 1 bis 6,
**dadurch gekennzeichnet,**
**dass** die Innenseite der Kappe (1) im Polbereich eine Vertiefung (11) aufweist, in die das Kopfteil (7) des Stifts (3) zumindest teilweise eingesetzt ist.

8. Femurkopfprothese nach einem der Ansprüche 1 bis 7,
**dadurch gekennzeichnet,**
**dass** die Innenseite der Kappe (1) einen sphärischen Bereich (23) und einen anschließenden zylindrischen Bereich (25) aufweist.

9. Femurkopfprothese nach einem der Ansprüche 1 bis 8,
**dadurch gekennzeichnet,**
**dass** die Kappe (1) im Randbereich wenigstens eine sich über einen Teil des Umfangs erstreckende Aussparung (27) aufweist.

10. Femurkopfprothese nach einem der Ansprüche 3 bis 9,
**dadurch gekennzeichnet,**
**dass** der als Knochenschraube ausgebildete Stift (3) an seiner Spitze einen Zapfen (19) aufweist.

11. Femurkopfprothese nach einem der Ansprüche 1 bis 10,
**dadurch gekennzeichnet,**
**dass** Kappe (1) und Stift (3) aus Metall gefertigt sind.

12. Femurkopfprothese nach einem der Ansprüche 1 bis 11,
**dadurch gekennzeichnet,**
**dass** die Innenseite der Kappe (1) mit Titan oder einer Titanlegierung beschichtet ist.

13. Fräswerkzeug zur Femurkopfbearbeitung für eine Femurkopfprothese nach einem der Ansprüche 1 bis 12, mit einem zentralen Führungsstift und wenigstens einer Schneide, welche zumindest bereichsweise entsprechend der Innenseite des sphärischen Bereichs (23) der Kappe (1) gekrümmt ist.

14. Fräswerkzeug nach Anspruch 13,
**dadurch gekennzeichnet ,**
**dass** mindestens zwei, insbesondere wenigstens drei zueinander in Drehrichtung versetzte Schneiden vorgesehen sind, die an ihren äußeren Enden über einen Ring miteinander verbunden sind.

15. Schneidstempel zur Femurkopfbearbeitung für eine Femurkopfprothese nach einem der Ansprüche 1 bis 12, mit einer ringförmigen Schneide vom Innendurchmesser eines Randbereichs der Kappe (1), wobei die Schneide einen bereichsweisen Unterbruch aufweist, welcher einer Aussparung (27) im Randbereich der Kappe (1) entspricht.
